# EUROPEAN PATENT SPECIFICATION

(11) **EP 4 499 091 B1**
(45) Date of publication and mention of the grant of the patent: **17.06.2026**
(21) Application number: 23714702.0
(22) Date of filing: 24.03.2023
(51) Int. Cl.: A61K 31/4184, A61P 25/02

(54) **USE OF N-(3-(4-(3-(DIISOBUTYLAMINO)PROPYL)PIPERAZIN-1-YL)PROPYL)-1H-BENZO[D]IMIDAZOL-2-AMINE SALTS AND SOLVATES THEREOF FOR THE TREATMENT OF SENSORY NEURON DISEASES**
VERWENDUNG VON N-(3-(4-(3-(DIISOBUTYLAMINO)PROPYL)PIPERAZIN-1-YL)PROPYL)-1H-BENZO[D]IMIDAZOL-2-AMINSALZEN UND SOLVATEN DAVON ZUR BEHANDLUNG SENSORISCHER NEURONENERKRANKUNGEN
UTILISATION DE SELS DE N-(3-(4-(3-(DIISOBUTYLAMINO)PROPYL)PIPÉRAZIN-1-YL)PROPYL)-1H-BENZO[D]IMIDAZOL-2-AMINE ET DE LEURS SOLVATES POUR LE TRAITEMENT DE MALADIES DES NEURONES SENSORIELS

(30) Priority: 25.03.2022 EP 22305364
(43) Date of publication of application: 05.02.2025
(73) Proprietor: Alzprotect, 59120 Loos (FR)
(72) Inventor: CALLIZOT, Noëlle, 13100 AIX-EN-PROVENCE (FR); ESTRELLA, Cécilia, 59800 LILLE (FR); VERWAERDE, Philippe, 59211 SANTES (FR); BRANTIS, Cyrille, 59700 MARCQ EN BAROEUL (FR); BURLET, Stéphane, 59170 CROIX (FR)
(74) Representative: Plasseraud IP
(86) International application number: PCT/EP2023/057659
(87) International publication number: WO 2023/180537

(56) References cited:
- WO-A1-2014/102339
- CALLIZOT N ET AL: "AZP2006, a new promising treatment for Alzheimer's and related diseases", SCIENTIFIC REPORTS, vol. 11, no. 1, 19 August 2021 (2021-08-19), XP002807452, ISSN: 2045-2322
- CELLA D ET AL: "Patient-reported peripheral neuropathy of doxorubicin and cisplatin with and without paclitaxel in the treatment of advanced endometrial cancer: Results from GOG 184", GYNECOLOGIC ONCOLOGY, ACADEMIC PRESS, LONDON, GB, vol. 119, no. 3, 1 December 2010 (2010-12-01), pages 538 - 542, XP027471663, ISSN: 0090-8258, [retrieved on 20100921], DOI: 10.1016/J.YGYNO.2010.08.022
- GISPEN W H ET AL: "ACTH/MSH like peptides in the treatment of cisplatin neuropathy", JOURNAL OF STEROID BIOCHEMISTRY & MOLECULAR BIOLOGY, ELSEVIER SCIENCE LTD., OXFORD, GB, vol. 43, no. 1-3, 1 September 1992 (1992-09-01), pages 179 - 183, XP025825080, ISSN: 0960-0760, [retrieved on 19920901], DOI: 10.1016/0960-0760(92)90205-W

## Description

The present invention is defined in the appended claims. It relates to novel uses of *N-(3-(4-(3-*(diisobutylamino)propyl)piperazin-1-yl)propyl)-1*H*-benzo[*d*]imidazol-2-amine salts and pharmaceutically acceptable solvates thereof, in the treatment and/or prevention of sensory neuron diseases.

### BACKGROUND OF THE INVENTION

Sensory neurons (SN) are neuronal cells present in the peripheral nervous system (PNS) which have diverse functions. The sensory components of the PNS consist of both the visceral sensory system which innervate the tympan, lungs, digestive and urinary tracts as well as several other organs; and the somatosensory system which relays the sensations of touch pain and other *stimuli* such as temperature and air motion. Different types of sensations are driven by neuron subtypes that are different with regards to innervation of the end organ, axon terminal morphology, intercellular interactions within the dorsal root ganglion (DRG) and the pattern of innervation of the spinal cord dorsal horn and the brain stem. SN comprise many axons adjacent to Schwann cells which could be either myelinating or non-myelinating. Myelin is a lipid rich insulating layer that surrounds axons, increases conduction velocity and decreases energy requirements for membrane repolarization. Myelinating Schwann cells wrap around axons of sensory neurons to form the protective myelin sheath. Schwann cells are also important as a cellular matrix and play a role for nervous conduction, nerve development and regeneration.

Sensory neuron diseases (SND) are peripheral nervous system diseases which lead to sensory neuron malfunction, degeneration and eventually apoptosis. As a result, senses of temperature, pain or vibration can be altered. SND include peripheral sensory neuronopathies or ganglionopathies which are peripheral neuropathies characterized by primary and selective degeneration of DRG T-shaped neurons and their projections, often resulting in a multifocal pattern of sensory deficits in the limbs. Certain SND primarily target the axon region and are thus referred to as peripheral axonopathies, they result in the axon degeneration according to a Wallerian process or an equivalent thereof. Additionally, if the axon is injured or destroyed, the Schwann cells cease to support the myelin sheath. Wallerian degeneration and the like further trigger the reprogramming of Schwann cells to a demyelinating phenotype, thus causing myelin loss. Certain SND involve a demyelination process and are referred to as peripheral myelinopathies.

The causes of SND include genetic predisposition, auto-immune disorders like Sjögren's syndrome and celiac disease as well as xenobiotics-related toxicity. Exposures to various neurotoxicants are responsible for the onset of toxic peripheral neuropathies which alter neurons, axons, Schwann cells and/or myelin. Indeed, chemicals such as arsenic, mercury or tin derivatives are compounds which improper handling remains a concern, especially in certain developing countries, because of their neurotoxic nature.

Another form of toxic peripheral neuropathy is chemotherapy-induced peripheral neuropathy (CIPN). CIPN is a common side-effect of many chemotherapy regimens. Indeed, cancer patients treated with platinum-based antineoplastic agents, vinca alkaloids, epothilones or taxanes often suffer from neuropathies caused by these molecules. Moreover, cancer survivors may require a lifetime of medical monitoring, in particular with platinum-based anticancer agents and taxanes, as CIPN may last several years following chemotherapy.

No specific treatment is available for sensory neurons diseases besides symptomatic drugs aimed at reducing pain and inflammation.

*N*-(3-(4-(3-(diisobutylamino)propyl)piperazin-1-yl)propyl)-1*H*-benzo[*d*]imidazol-2-amine sulphate salts and solvates thereof, previously disclosed in WO 2014/102339 are useful for the treatment and/or prevention of neurodegenerative diseases, amyloidopathies, tauopathies and developmental disease. These compounds are especially of interest for Alzheimer's disease, Parkinson's disease and tauopathies: a phase 2A clinical trial with *N*-(3-(4-(3-(diisobutylamino)propyl)piperazin-1-yl)propyl)-1*H*-benzo[*d*]imidazol-2-amine di-sulphate salt for patients suffering from progressive supranuclear palsy (PSP) is ongoing.

These salts are able to modulate Tau phosphorylation and increase progranulin (PGRN) neurotrophic factor levels which deficiency is known to accelerate Tau deposition and phosphorylation, as evidenced in human tau-expressing mice (J. Neuropathol. Exp. Neurol. 74, 158-165 (2015)). Therefore, these compounds are especially useful for the treatment of tauopathies. Cella D. eta 1. (Gynecologic Oncology (2010), 119(3), 538 - 542) and Gispen W.H. et al. (Journal of Steroid Biochemistry & Molecular Biology (1992), 43(1-3), 179 - 183) disclose compounds useful for the treatment of peripheral neuropathy induced by doxorubicin or cisplatin.

As there is still a need in the art for chemical entities that could be used in the treatment or prophylaxis of sensory neuron diseases, the Applicant investigated the potential of using salts of *N-*(3-(4-(3-(diisobutylamino)propyl)piperazin-1-yl)propyl)-1*H*-benzo[d]imidazol-2-amine and solvates thereof for such purpose.

### SUMMARY OF THE INVENTION

The present invention is based on the unexpected findings that salts of *N-*(3-(4-(3-(diisobutylamino)propyl)piperazin-1-yl)propyl)-1*H*-benzo[d]imidazol-2-amine have neuroprotective effects on sensory neurons.

The invention thus concerns salts of *N*-(3-(4-(3-(diisobutylamino)propyl)piperazin-1-yl)propyl)-1*H*-benzo[*d*]imidazol-2-amine and solvates thereof for use in the treatment and/or prevention of sensory neuron diseases.

### DETAILED DESCRIPTION OF THE INVENTION

Applicant has shown that salts of *N*-(3-(4-(3-(diisobutylamino)propyl)piperazin-1-yl)propyl)-1*H*-benzo[*d*]imidazol-2-amine and pharmaceutically acceptable solvates thereof exert beneficial effects on primary culture of sensory neurons and Schwann cells by promoting their survival and the integrity of their neurite network after chemotherapy agent-induced damages.

All these findings support that salts of *N*-(3-(4-(3-(diisobutylamino)propyl)piperazin-1-yl)propyl)-1*H*-benzo[*d*]imidazol-2-amine and pharmaceutically acceptable solvates thereof could be an effective therapy to improve sensory neuron diseases and thus useful as a medicament, in particular for treating or preventing sensory neuron diseases.

Sensory neuron diseases include, but are not limited to neuropathies caused by Sjögren's syndrome, celiac neuropathies, toxic peripheral neuropathies and chemotherapy induced peripheral neuropathies (CIPN).

Sjögren's syndrome is a heterogeneous auto-immune inflammatory disorder frequently involving peripheral nerves with a wide spectrum of sensory modalities and distribution patterns. Neuropathies caused by Sjögren syndrome may be neuronopathies, axonopathies and/or myelinopathies (Seeliger et al. Frontiers in Immunology 2019, Vol. 10, Article 1600).

Celiac neuropathies refer to neuronopathies, chronic inflammatory demyelinating neuropathies, autonomic neuropathies and mononeuritis multiplex, all caused by celiac disease. Celiac disease is an autoimmune gastrointestinal disease in which damage to the inner lining of the small intestine prevents sufficient absorption of the essential proteins, fats, carbohydrates, vitamins, and minerals from food.

Toxic peripheral neuropathies designate the neuropathies caused by exposure to chemical neurotoxicants. Toxic peripheral neuropathies comprise toxic peripheral axonopathies, especially those caused by hexane, 2-hexanone, carbon disulfide and 1-bromopropane, toxic peripheral myelinopathies, especially those caused by hexachlorophene, triethyltin, tellurium, diphteria toxin, disulfiram, *N,N-*diethyldithiocarbamate, lysolecithin, ethidium bromide, toxic peripheral neuronopathies, especially those caused by methylmercury and catecholamines.

Chemotherapy-induced peripheral neuropathies (CIPN) designate the peripheral neuropathies caused by common chemotherapeutic agents, including platinum-based antineoplastic agents, vinca alkaloids, epothilones such as ixabepilone, taxanes, proteasome inhibitors such as bortezomib and carfilzomib, and immunomodulatory drug thalidomide. CIPN is the major reason for stopping the anti-tumor therapy or changing the dose regimen. CIPN is partly reversible but damages could be irreversible in the worst-case scenario. More than half of the patients treated with antimitotic drugs describe paresthesia and dysesthesia, which appear 24-72 h after administration. There is no treatment on the market to prevent CIPN. CIPN preferentially take place in dorsal root ganglia (DRG), sensory neurons, satellite cells and Schwann cells (SCs). CIPN comprise chemotherapy-induced axonopathies caused by taxanes such as paclitaxel and docetaxol, vinca alkaloids such as vinblastine, vinfluine, and vinorelbine, chemotherapy-induced neuronopathies caused by platinum-based antineoplastic agents such as cisplatin, oxaliplatin and carboplatin.

In one embodiment, the invention concerns salts of *N-(3-(4-(3-*(diisobutylamino)propyl)piperazin-1-yl)propyl)-1*H*-benzo[*d*]imidazol-2-amine or a pharmaceutically acceptable solvate thereof as defined herein for use in treating and/or preventing sensory neuron diseases selected from toxic peripheral neuropathies and chemotherapy-induced peripheral neuropathy (CIPN). Preferably the disease is selected from toxic peripheral neuronopathies, chemotherapy-induced axonopathies caused by taxanes and vinca alkaloids, chemotherapy-induced neuronopathies caused by platinum-based antineoplastic agents. More preferably the disease is selected from toxic peripheral neuronopathies, chemotherapy-induced neuronopathies caused by platinum-based antineoplastic agents. More preferably the disease is selected from chemotherapy-induced neuronopathies caused by platinum-based antineoplastic agents. Even more preferably the disease is selected from chemotherapy-induced neuronopathies caused by platinum-based antineoplastic agents.

In other terms, the invention also provides for the claimed compound for use in a method of treating and/or preventing a sensory neuron disease, in particular those cited above as well as embodiments thereof, comprising administering to a patient in need thereof a pharmaceutically effective amount of a salt of *N*-(3-(4-(3-(diisobutylamino)propyl)piperazin-1-yl)propyl)-1*H*-benzo[*d*]imidazol-2-amine or a pharmaceutically acceptable solvate thereof as described herein. In a particular embodiment, the disease is selected from toxic peripheral neuronopathies, chemotherapy-induced neuronopathies caused by platinum-based antineoplastic agents.

In one particular embodiment, the invention also concerns salts of *N*-(3-(4-(3-(diisobutylamino)propyl)piperazin-1-yl)propyl)-1*H*-benzo[*d*]imidazol-2-amine or a pharmaceutically acceptable solvate thereof as defined herein for use in delaying in a patient the onset of sensory neuron diseases, in particular selected from toxic peripheral neuropathies and chemotherapy-induced peripheral neuropathy (CIPN). Preferably the disease is selected from toxic peripheral neuronopathies, chemotherapy-induced peripheral neuropathy. (CIPN). More preferably the disease is selected from toxic peripheral neuronopathies, chemotherapy-induced axonopathies caused by taxanes and vinca alkaloids, chemotherapy-induced neuronopathies caused by platinum-based antineoplastic agents. More preferably the disease is selected from toxic peripheral neuronopathies, chemotherapy-induced neuronopathies caused by platinum-based antineoplastic agents. More preferably the disease is selected from chemotherapy-induced neuronopathies caused by platinum-based antineoplastic agents. Even more preferably the disease is selected from chemotherapy-induced neuronopathies caused by platinum-based antineoplastic agents.

In other terms, the invention provides for the claimed compound for use in a method for delaying in a patient the onset of sensory neuron diseases, in particular those cited above as well as embodiments thereof, comprising administering to a patient in need thereof a pharmaceutically effective amount of a salt of *N*-(3-(4-(3-(diisobutylamino)propyl)piperazin-1-yl)propyl)-1*H*-benzo[d]imidazol-2-amine salt or a pharmaceutically acceptable solvate thereof. In a particular embodiment, the disease is selected from toxic peripheral neuronopathies, chemotherapy-induced neuronopathies caused by platinum-based antineoplastic agents.

According to one embodiment, the salts of *N*-(3-(4-(3-(diisobutylamino)propyl)piperazin-1-yl)propyl)-1*H*-benzo[*d*]imidazol-2-amine, as well as their pharmaceutical acceptable solvates may be administered as part of a combination therapy. Thus, are included within the scope of the present invention embodiments comprising coadministration of compositions and medicaments which contain, in addition to a salt of *N*-(3-(4-(3-(diisobutylamino)propyl)piperazin-1-yl)propyl)-1*H*-benzo[d]imidazol-2-amine or a pharmaceutically acceptable solvate thereof as active ingredient, additional therapeutic agents and/or active ingredients. Such multiple drug regimens, often referred to as "combination therapy", may be used in the treatment and/or prevention of any sensory neuron disease. The use of such combinations of therapeutic agents is especially pertinent with respect to the treatment of the above-mentioned sensory neuron diseases within a patient in need of treatment or one at risk of becoming such a patient.

In addition to the requirement of therapeutic efficacy, which may necessitate the use of active agents in addition to the salts of *N*-(3-(4-(3-(diisobutylamino)propyl)piperazin-1-yl)propyl)-1*H*-benzo[*d*]imidazol-2-amine or pharmaceutically acceptable solvates thereof, there may be additional rationales which compel or highly recommend the use of combinations of drugs involving active ingredients which represent adjunct therapy, *i.e.,* which complement and supplement the function performed by the salts of *N*-(3-(4-(3-(diisobutylamino)propyl)piperazin-1-yl)propyl)-1*H*-benzo[d]imidazol-2-amine or pharmaceutically acceptable solvates thereof. Suitable supplementary therapeutic agents used for the purpose of auxiliary treatment include drugs which, instead of directly treating and/or preventing a disease or condition mediated by or associated with sensory neuron degeneration, treat diseases or conditions which directly result from or indirectly accompany said degenerations.

According to a further feature of the present invention, a salt of *N-*(3-(4-(3-(diisobutylamino)propyl)piperazin-1-yl)propyl)-1*H*-benzo[*d*]imidazol-2-amine, a pharmaceutically acceptable solvate thereof may be used in combination therapy with other drugs. More particularly, the compound of Formula I or Formula II, as well as pharmaceutically acceptable solvates thereof, may be used as an adjunct therapy in combination with chemotherapeutic agents, including platinum-based antineoplastic agents such as cisplatin, oxaliplatin and carboplatin, vinca alkaloids such as vinblastine, vinfluine, and vinorelbine, epothilones such as ixabepilone, taxanes such as paclitaxel and docetaxol, proteasome inhibitors such as bortezomib and carfilzomib, and immunomodulatory drug thalidomide.

Thus, the methods of treatment and pharmaceutical compositions of the present invention may employ a salt of *N*-(3-(4-(3-(diisobutylamino)propyl)piperazin-1-yl)propyl)-1*H-*benzo[*d*]imidazol-2-amine or a pharmaceutically acceptable solvate thereof in monotherapy of sensory neuron diseases. However, said methods and compositions may also be used in multiple therapy in which one or more N-(3-(4-(3-(diisobutylamino)propyl)piperazin-1-yl)propyl)-1*H-*benzo[*d*]imidazol-2-amine salts or their pharmaceutically acceptable solvates are co-administered in combination with one or more other therapeutic agents.

In the above-described embodiment, combinations of a salt of *N*-(3-(4-(3-(diisobutylamino)propyl)piperazin-1-yl)propyl)-1*H*-benzo[d]imidazol-2-amine or a pharmaceutically acceptable solvate thereof and other therapeutic active agents may be administered, in terms of dosage forms, either separately or in conjunction with each other, and in terms of their time of administration, either serially or simultaneously. Thus, the administration of one component agent may be prior to, concurrent with, or subsequent to the administration of the other component agent(s).

Generally, for pharmaceutical use, the salts of *N-*(3-(4-(3-(diisobutylamino)propyl)piperazin-1-yl)propyl)-1*H*-benzo[*d*]imidazol-2-amine or pharmaceutically acceptable solvates thereof may be formulated as a pharmaceutical composition comprising at least one salt of *N*-(3-(4-(3-(diisobutylamino)propyl)piperazin-1-yl)propyl)-1*H*-benzo[*d*]imidazol-2-amine or a pharmaceutically acceptable solvate thereof and at least one pharmaceutically acceptable carrier, diluent, excipient and/or adjuvant, and optionally one or more further therapeutic agents and/or active ingredients.

By means of non-limiting examples, pharmaceutical composition may be in a dosage form suitable for oral administration, for parenteral administration (such as by intravenous, intramuscular or subcutaneous injection or intravenous infusion), for topical administration, for administration by inhalation, by a skin patch, by an implant, by a suppository, *etc.* Such suitable administration forms - which may be solid, semi-solid or liquid, depending on the manner of administration - as well as methods and carriers, diluents and excipients for use in the preparation thereof, will be clear to the skilled person; reference is made to the latest edition of Remington's Pharmaceutical Sciences. The pharmaceutical compositions may be formulated in solid form and re-dissolved or suspended prior to use. Preferred pharmaceutical compositions of the succinate salts of the invention or solvates thereof are in a solid dosage form suitable for oral administration.

Some preferred, but non-limiting examples of dosage forms include tablets, pills, powders, lozenges, sachets, cachets, elixirs, suspensions, emulsions, solutions, syrups, aerosols, ointments, cremes, lotions, soft and hard gelatin capsules, suppositories, drops, sterile injectable solutions and sterile packaged powders (which are usually reconstituted prior to use) for administration as a bolus and/or for continuous administration, which may be formulated with carriers, excipients, and diluents that are suitable *per se* for such formulations, such as lactose, dextrose, sucrose, sorbitol, mannitol, starches, agar, gum acacia, calcium phosphate, alginates, tragacanth, gelatin, calcium silicate, microcrystalline cellulose, polyvinylpyrrolidone, polyethylene glycol, cellulose, (sterile) water, methylcellulose, methyl- and propylhydroxybenzoates, talc, magnesium stearate, edible oils, vegetable oils and mineral oils or suitable mixtures thereof. Particularly preferred dosage forms of the succinate salts of the invention and solvates thereof are soft and hard capsules, in particular soft and hard gelatin capsules, preferably hard gelatin capsules, which are formulated with at least one microcrystalline cellulose excipient, especially Avicel^{®} PH101. The pharmaceutical compositions can optionally contain other substances that are commonly used in pharmaceutical formulations, such as lubricating agents, wetting agents, emulsifying and suspending agents, dispersing agents, disintegrating agents, stabilizing agents, isotonic agents, bulking agents, fillers, preserving agents, sweetening agents, flavouring agents, perfuming agents, colouring agents, antibacterial agents and/or antifungal agents such as parabens, chlorobutanol, phenol, sorbic acid, dispensing agents, flow regulators, release agents, *etc.* The compositions may also be formulated so as to provide rapid, sustained or delayed release of the active compound(s) contained therein.

The pharmaceutical compositions of the invention are preferably in a unit dosage form, and may be suitably packaged, for example in a box, blister, vial, bottle, sachet, ampoule or in any other suitable single-dose or multi-dose holder or container (which may be properly labeled); optionally with one or more leaflets containing product information and/or instructions for use. Generally, such unit dosages will contain between 0.05 and 1000 mg, and usually between 1 and 500 mg, of the at least one compound of the invention, e.g. about 10, 25, 50, 100, 200, 300 or 400 mg per unit dosage.

Usually, depending on the condition to be prevented or treated and the route of administration, the active compound of the invention will usually be administered between 0.01 to 100 mg per kilogram, more often between 0.1 and 50 mg, such as between 1 and 25 mg, for example about 0.5, 1, 2, 5, 10, 15, 20 or 25 mg, per kilogram body weight of the patient per day, which may be administered as a single daily dose, divided over one or more daily doses, or essentially continuously, e.g. using a drip infusion.

In one embodiment, the salts of N-(3-(4-(3-(diisobutylamino)propyl)piperazin-1-yl)propyl)-1H-benzo[d]imidazol-2-amine are N-(3-(4-(3-(diisobutylamino)propyl)piperazin-1-yl)propyl)-1H-benzo[d]imidazol-2-amine sulphate salts and pharmaceutically acceptable solvates thereof.

In one preferred embodiment, the sulphate salts of *N-(3-(4-(3-*(diisobutylamino)propyl)piperazin-1-yl)propyl)-1H-benzo[d]imidazol-2-amine are those of Formula I 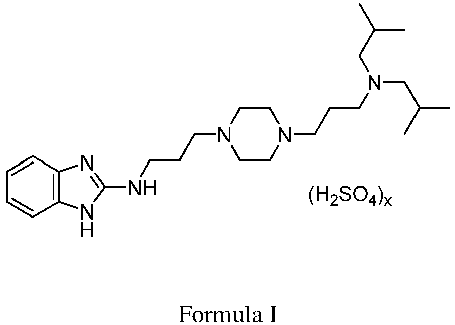 wherein x is 0.5 to 4, preferably x is 0.5 to 3.5, more preferably x is 0.9 to3.

In other terms, the sulphate salt of N-(3-(4-(3-(diisobutylamino)propyl)piperazin-1-yl)propyl)-1*H*-benzo[*d*]imidazol-2-amine contains 0.5 to 4 equivalents, preferably 0.5 to 3.5 equivalents, more preferably 0.9 to 3 equivalents of sulphate for one molecule of *N-(3-(4-(3-*(diisobutylamino)propyl)piperazin-1-yl)propyl)-1*H*-benzo[*d*]imidazol-2-amine.

In one preferred embodiment, x is 1.7 to 2.3, preferably x is 1.9 to 2.1, more preferably x is about 2 or x is 2,
In one particularly preferred embodiment, the sulphate salt is *N*-(3-(4-(3-(diisobutylamino)propyl)piperazin-1-yl)propyl)-1*H-*benzo[d]imidazol-2-amine di-sulphate.

In one embodiment, the sulphate salt of Formula I is in the form of a pharmaceutically acceptable solvate, preferably a hydrate. The solvate stoichiometry is between 0.5 to 5, preferably between 1 to 4, more preferably between 1.5 to 2.5, still more preferably between 1.8 to 2.2, even more preferably 2 or about 2 molecules of solvate for 1 molecule of sulphate salt of Formula I.

In another embodiment, the salts of *N*-(3-(4-(3-(diisobutylamino)propyl)piperazin-1-yl)propyl)-1*H*-benzo[d]imidazol-2-amine are *N*-(3-(4-(3-(diisobutylamino)propyl)piperazin-1-yl)propyl)-1*H*-benzo[d]imidazol-2-amine succinate salts and pharmaceutically acceptable solvates thereof.

In one preferred embodiment, the succinate salts of *N*-(3-(4-(3-(diisobutylamino)propyl)piperazin-1-yl)propyl)-1*H*-benzo[*d*]imidazol-2-amine are those of Formula II 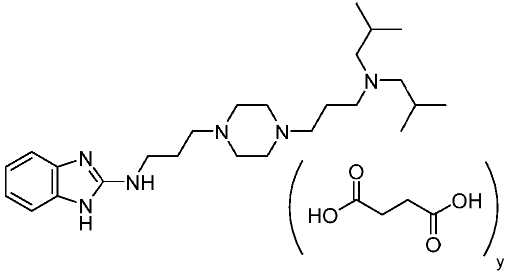 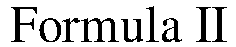 wherein **y** is 1 to 4, preferably **y** is 1.4 to 3.1, more preferably y is 1.4 to 1.6, still more preferably **y** is about 1.5, even more preferably **y** is 1.5.

In other words, the succinate salt of *N*-(3-(4-(3-(diisobutylamino)propyl)piperazin-1-yl)propyl)-1*H*-benzo[d]imidazol-2-amine contains 1 to 4 equivalents, preferably 1.4 to 3.1 equivalents, more preferably 1.4 to 1.6 equivalents, still more preferably about 1.5 equivalents and even more preferably 1.5 equivalents of succinate for one molecule of *N-*(3-(4-(3-(diisobutylamino)propyl)piperazin-1-yl)propyl)-1*H*-benzo[*d*]imidazol-2-amine.

In one preferred embodiment, the succinate salt of Formula II is the sesqui-succinate salt of *N*-(3-(4-(3-(diisobutylamino)propyl)piperazin-1-yl)propyl)-1H-benzo[d]imidazol-2-amine.

In one embodiment, preferred succinate salts are the compounds of Formula II wherein **x** is 2.9 to 3.1, preferably wherein **x** is about 3, more preferably **x** is 3.

In another preferred embodiment, the succinate salt is the tris-succinate salt of *N-*(3-(4-(3-(diisobutylamino)propyl)piperazin-1-yl)propyl)-1*H*-benzo[*d*]imidazol-2-amine.

In a particular embodiment, the succinate salt of Formula II is in the form of a pharmaceutically acceptable solvate, preferably a hydrate. The solvate stoichiometry is between 0.4 to 2, preferably between 0.4 to 1.2, more preferably between 0.5 to 1.1, still more preferably about 0.5 or about 1.1, even more preferably 0.5 or 1.1 molecules of solvate for 1 molecule of succinate salt of Formula II.

In one embodiment, the succinate salt of Formula II is *N*-(3-(4-(3-(diisobutylamino)propyl)piperazin-1-yl)propyl)-1*H*-benzo[*d*]imidazol-2-amine sesqui-succinate salt hemi-hydrate.

In another embodiment, the succinate salt of Formula II is *N*-(3-(4-(3-(diisobutylamino)propyl)piperazin-1-yl)propyl)-1*H*-benzo[d]imidazol-2-amine sesqui-succinate salt mono-hydrate.

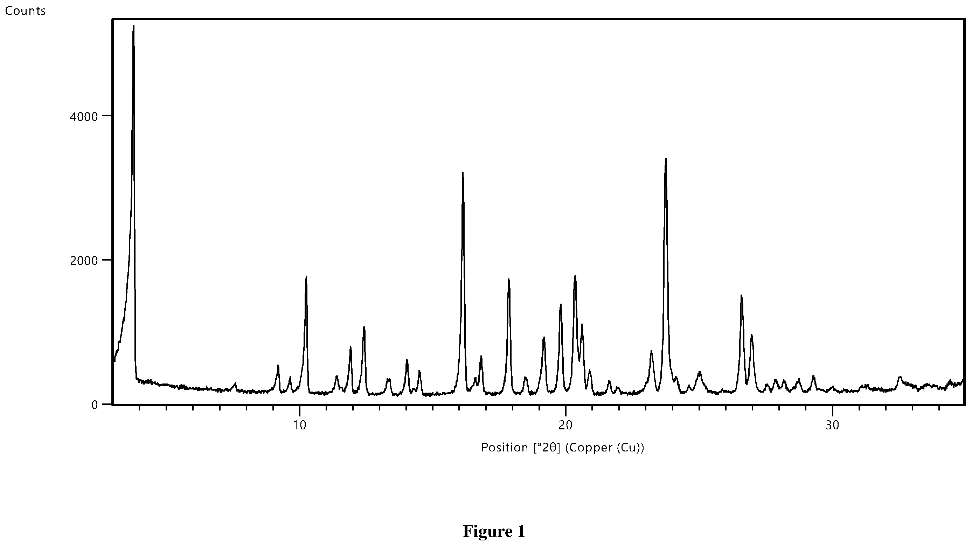

In one embodiment, one particularly preferred succinate salt is the compound of Formula II wherein y is 1.5 under the crystalline form having an X-ray powder diffraction (XRPD) pattern comprising peaks at diffraction angles of 2θ=3.8° ±0.2°, 10.3° ±0.2°, 12.4° ±0.2°, 16.2° ±0.2°, 17.9° ±0.2°, 19.8° ±0.2°, 20.4° ±0.2°, 23.8° ±0.2° and 26.7° ±0.2° when irradiated with a CuKα light source. This specific crystalline form is referred to as "Form 1" throughout the application and its XRPD diffractogram recorded on a PANalytical X'pert pro with PIXcel detector (128 channels) is shown in Figure 1.

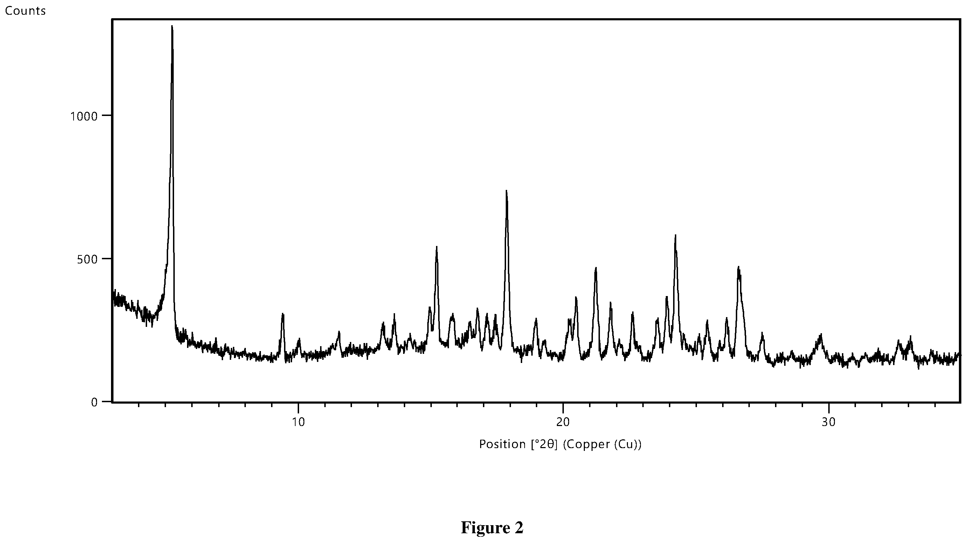

Another preferred succinate salt is the compound of Formula II wherein y is 3 under the crystalline form having an X-ray powder diffraction (XRPD) pattern comprising peaks at diffraction angles of 2θ=5.3° ±0.2°, 15.0° ±0.2°, 15.3° ±0.2°, 16.8° ±0.2°, 17.9° ±0.2°, 20.5° ±0.2°, 21.2° ±0.2°, 23.9° ±0.2°, 24.3° ±0.2° and 26.6° ±0.2° when irradiated with a CuKα light source. This specific crystalline form is referred to as "Form 2" throughout the application and its XRPD diffractogram recorded on a PANalytical X'pert pro with PIXcel detector (128 channels) is shown in Figure 2.

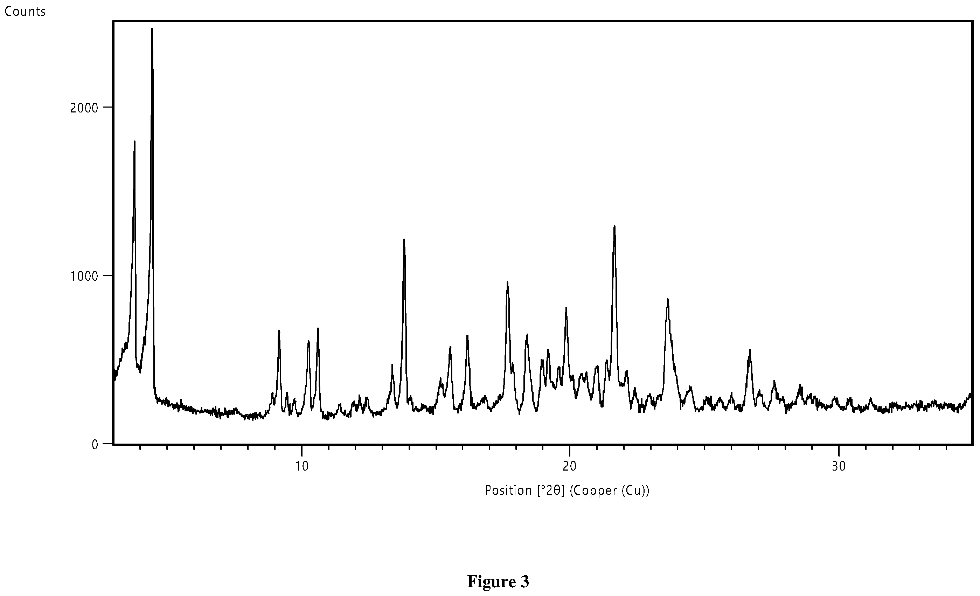

Another preferred succinate salt is the hemi-hydrate of the compound of Formula II wherein y is 1.5, and under the crystalline form having an X-ray powder diffraction (XRPD) pattern comprising peaks at diffraction angles of 2θ=3.8° ±0.2°, 4.4° ±0.2°, 9.2° ±0.2°, 10.6° ±0.2°, 13.8° ±0.2°, 17.7° ±0.2°, 19.9° ±0.2°, 21.6° ±0.2° and 23.6° ±0.2° when irradiated with a CuKα light source. Its XRPD diffractogram recorded on a PANalytical X'pert pro with PIXcel detector (128 channels) is shown in Figure 3.

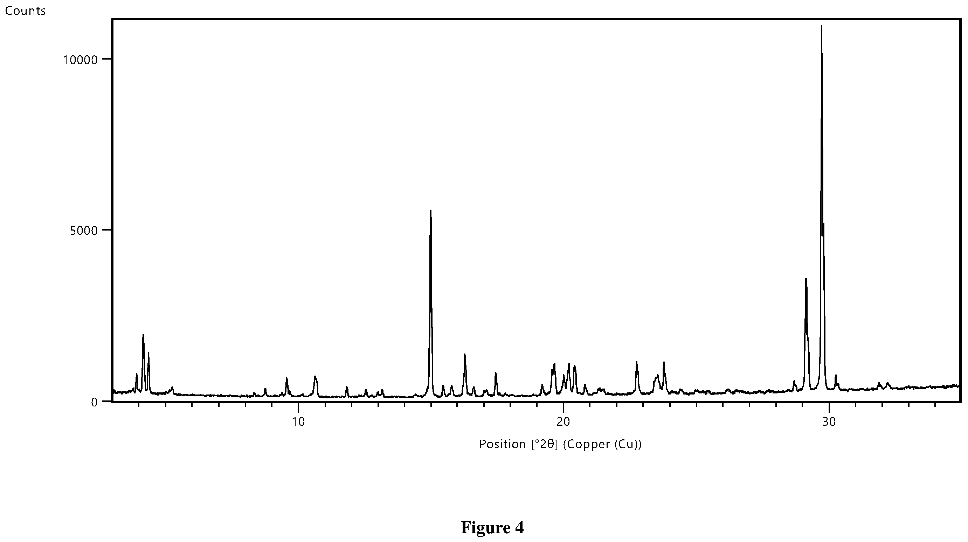

Another preferred succinate salt is the mono-hydrate compound of Formula I wherein y is 1.5, and under the crystalline form having an X-ray powder diffraction (XRPD) pattern comprising peaks at diffraction angles of 2θ=4.2 ±0.2°, 4.4° ±0.2°, 9.6° ±0.2°, 10.6° ±0.2°, 15.0° ±0.2°, 16.3° ±0.2°, 17.4° ±0.2°, 19.7° ±0.2°, 22.8° ±0.2°, 29.1 and 29.7° ±0.2° when irradiated with a CuKα light source. Its XRPD diffractogram recorded on a PANalytical X'pert pro with PIXcel detector (128 channels) is shown in Figure 4.

All references to compounds of Formula I and Formula II include references to solvates, in particular hydrates, multi- component complexes and liquid crystals thereof.

The compounds disclosed throughout the present application were named using ChemDraw^{®} Ultra version 11.0 (CambridgeSoft, Cambridge, MA, USA).

*N*-(3-(4-(3-(diisobutylamino)propyl)piperazin-1-yl)propyl)-1*H*-benzo[d]imidazol-2-amine free base can be obtained as disclosed in WO 2006/051489. The sulphate salts can be obtained as disclosed in WO 2014/102339. The succinate salts and solvates thereof can be prepared according to techniques known in the art such as those involving precipitation, crystallization, recrystallization, lyophilisation, phase transfer or ion exchange resins.

### DEFINITIONS

The definitions and explanations below are for the terms as used throughout the entire application, including both the specification, the figures and the claims.

Unless otherwise stated any reference to compounds of the invention herein, means the compound of Formula I or II as such as well as its pharmaceutically acceptable solvates. All references to compounds of Formula I or II include references to pharmaceutically acceptable solvates thereof and all crystalline forms thereof.

The term "administration", or a variant thereof (e.g."administering"), means providing the active agent or active ingredient (e.g. *N*-(3-(4-(3-(diisobutylamino)propyl)piperazin-1-yl)propyl)-1*H*-benzo[*d*]imidazol-2-amine), alone or as part of a pharmaceutically acceptable composition, to the patient in whom/which the condition, symptom, or disease is to be treated or prevented.

The term "human" refers to a subject of both genders and at any stage of development (*i.e.* neonate, infant, juvenile, adolescent, adult).

The term "patient" refers to a warm-blooded animal, more preferably a human, who/which is awaiting the receipt of, or is receiving medical care or is/will be the object of a medical procedure.

By "pharmaceutically acceptable" is meant that the ingredients of a pharmaceutical composition are compatible with each other and not deleterious to the patient thereof.

The terms "prevent", "preventing" and "prevention", as used herein, refer to a method of delaying or precluding the onset of a condition or disease and/or its attendant symptoms, barring a patient from acquiring a condition or disease, or reducing a patient's risk of acquiring a condition or disease.

The expression "reducing" as used herein refers to a partial reduction or a complete reduction.

The term "sesqui" as used herein, refers to one and a half equivalent, a sesqui-succinate salt of a compound means its succinic acid addition salt wherein the succinic acid is present in a ratio of 3:2 compared with the compound in its free base form.

The term "solvate" is used herein to describe a compound in this invention that contains stoichiometric or sub-stoichiometric amounts of one or more pharmaceutically acceptable solvent molecule such as ethanol. The term "hydrate" is employed when said solvent is water. The pharmaceutically acceptable solvent molecules may be co-crystallized with the compound of the invention, and/or be present in crystalline and/or amorphous phases of solids thereof, and/or be adsorbed thereto.

The term "therapeutically effective amount" (or more simply an "effective amount") as used herein means the amount of active agent or active ingredient (e.g. *N*-(3-(4-(3-(diisobutylamino)propyl)piperazin-1-yl)propyl)-1*H*-benzo[*d*]imidazol-2-amine) that is sufficient to achieve the desired therapeutic or prophylactic effect in the patient to which/whom it is administered.

The terms "treat", "treating" and "treatment, as used herein, are meant to include alleviating, attenuating or abrogating a condition or disease and/or its attendant symptoms.

The present invention will be better understood with reference to the following examples.

### BRIEF DESCRIPTION OF THE DRAWINGS

**Figure 1** shows the XRPD 2Θ diffractogram of Form 1, crystalline form of *N*-(3-(4-(3-(diisobutylamino)propyl)piperazin-1-yl)propyl)-1H-benzo[d]imidazol-2-amine sesqui-succinate salt.
**Figure 2** shows the XRPD 2Θ diffractogram of Form 2, crystalline form of *N-*(3-(4-(3-(diisobutylamino)propyl)piperazin-1-yl)propyl)-1H-benzo[d]imidazol-2-amine tris-succinate salt.
**Figure 3** shows the XRPD 2Θ diffractogram of a crystalline form of *N-*(3-(4-(3-(diisobutylamino)propyl)piperazin-1-yl)propyl)-1H-benzo[d]imidazol-2-amine sequi-succinate salt hemi-hydrate (0.5 eq. of water).
**Figure 4** shows the XRPD 2Θ diffractogram of a crystalline form of *N*-(3-(4-(3-(diisobutylamino)propyl)piperazin-1-yl)propyl)-1*H*-benzo[d]imidazol-2-amine sesqui-succinate salt mono-hydrate (1.1 eq. of water).
**Figure 5** shows the ¹H-NMR spectrum of Form 1, crystalline form of *N*-(3-(4-(3-(diisobutylamino)propyl)piperazin-1-yl)propyl)-1*H*-benzo[*d*]imidazol-2-amine sesqui-succinate salt (DMSO-d₆, 500.12 MHz).
**Figure 6** shows the ¹³C Quant-NMR spectrum of Form 1, crystalline form of *N*-(3-(4-(3-(diisobutylamino)propyl)piperazin-1-yl)propyl)-1*H*-benzo[*d*]imidazol-2-amine sesqui-succinate salt (DMSO-d₆, 500.12 MHz).
**Figure 7** shows the effect of an incubation of *N*-(3-(4-(3-(diisobutylamino)propyl)piperazin-1-yl)propyl)-1*H*-benzo[*d*]imidazol-2-amine) di-sulphate for 24h with cisplatin injury, in a primary culture of rat sensory neurons and Schwann cells on survival (A) and neurite network (B) of MAP-2 positive SNs. Results are expressed as a percentage of control as mean ± SEM (n=4-6/group). One-way ANOVA followed by PLSD Fisher's test.
**Figure 8** shows the effect of an incubation of *N-*(3-(4-(3-(diisobutylamino)propyl)piperazin-1-yl)propyl)-1*H*-benzo[*d*]imidazol-2-amine) sesqui-succinate salt Form 1 for 24h with cisplatin injury, in a primary culture of rat sensory neurons and Schwann cells on survival (A) and neurite network (B) of MAP-2 positive SNs. Results are expressed as a percentage of control as mean ± SEM (n=5-6/group). One-way ANOVA followed by PLSD Fisher's test.

### CHEMISTRY EXAMPLES

The following abbreviations are used throughout the present application: °C: Celsius degrees, DMSO: dimethylsulfoxyde, δ: NMR chemical shifts expressed in ppm, eq: equivalent(s), EDTA: ethylenediamine tetra-acetic acid, eq.: equivalent(s), g: gram(s), h: hour(s), HPLC: high performance liquid chromatography, Hz: Hertz, L: liter(s), M: mol/L, mM: mmol/L, µM: µmol/L, Me: methyl, MEK: methyl ethyl ketone, mg: milligram(s), MHz: megahertz, min: minute(s), mL: milliliter(s), mm: millimeter(s), mol: mole(s), mmol: millimole(s), µg: microgram(s), µmol: micromole(s), nm: nanometer(s), NMR: nuclear magnetic resonance, PBS: phosphate-buffered saline, PGRN: progranulin, ppm: party per million, RH: relative humidity, RPM: round(s) per minute, rt: retention time, RT: room temperature (*ca* 15-25 °C), s: second(s), TFA: trifluoroacetic acid, UV: ultraviolet, v: volume, XRPD: X-ray powder diffraction.

All reported temperatures are expressed in degrees Celsius (°C); all reactions were carried out at room temperature (RT) unless otherwise stated.

Experimental set-up or purification procedures that were used in this invention, when not described in specific details, are assumed to be known to those conversant in the art and are described in such standard reference manuals such as: i) Gordon, A. J.; Ford, R. A. "The Chemist's Companion - A Handbook of Practical Data, Techniques, and References", Wiley: New York, 1972; ii) Vogel's Textbook of Practical Organic Chemistry, Pearson Prentice Hall: London, 1989; iii) P. Heinrich Stahl and Camille G. Wernuth 'Handbook of Pharmaceutical Salts', Wiley VCH.

### HPLC analysis.

| | |
|---|---|
| Instrument: | Dionex Ultimate 3000 |
| Column: | Supelco Ascentis Express C18 15 x 4.6 mm 2.7 µm |
| Column Temperature: | 50 °C |
| Autosampler Temperature: | Ambient |
| UV wavelength: | 275 nm |
| Injection Volume: | 20 µL |
| Flow Rate: | 1 mL/min |
| Mobile Phase A: | 0.1 % TFA in Water |
| Mobile Phase B: | 0.1 % TFA in Acetonitrile |

### Gradient program:

| **Time (minutes)** | **Solvent B [%]** |
|---|---|
| 0.0 | 5 |
| 30.0 | 60 |
| 33.0 | 60 |
| 33.1 | 5 |
| 40.0 | 5 |

### NMR analysis

NMR experiments were performed on a Bruker AVIIIHD spectrometer equipped with a DCH cryoprobe operating at 500.12 MHz for protons and carbons. Experiments were performed in deuterated DMSO and each sample was prepared to *ca.* 10 mM concentration. Further analysis was optionally performed in D₂O and/or methanol-d₄. Chemical shifts are expressed in parts per million, (ppm, δ units). Coupling constants are expressed in Hz. Abbreviations for multiplicities observed in NMR spectra are as follows: s (singlet), d (doublet), t (triplet), q (quadruplet), m (multiplet), br (broad).

### X-ray Powder Diffraction (XRPD)

XRPD analysis was carried out on a PANalytical X'pert pro with PIXcel detector (128 channels), scanning the samples between 3 and 35° 2θ. The material was gently ground to release any agglomerates and loaded onto a multi-well plate with Mylar polymer film to support the sample. The multi-well plate was then placed into the diffractometer and analysed using Cu Kα radiation (α₁ λ = 1.54060 Å; α₂ = 1.54443 Å; β = 1.39225 Å; α₁ : α₂ ratio = 0.5) running in transmission mode (step size 0.0130° 2θ, step time 18.87s) using 40 kV / 40 mA generator settings. Data were visualized and images generated using the HighScore Plus 4.7 desktop application (PANalytical, 2017).

Solvents and reagents were purchased and used as received from commercial vendors unless otherwise specified.

Example 1: synthesis and characterisation of Form 1: crystalline form of *N-(3-(4-(3-*(diisobutylamino)propyl)piperazin-1-yl)propyl)-1*H*-benzo[*d*]imidazol-2-amine sesqui-succinate salt 514.6 mg of *N*-(3-(4-(3-(diisobutylamino)propyl)piperazin-1-yl)propyl)-1*H*-benzo[*d*]imidazol-2-amine was weighed out in a 20 mL scintillation vial. 10 mL of methyl ethyl ketone (99% pure) was added to the vial which was stirred at 40 °C using a temperature-controlled block, a stirrer plate, and magnetic needle. No clear solution was observed so further 5 mL of MEK was added to aid dissolution at 40°C. A clear solution was obtained; the clear solution at 40°C was polish-filtered using PTFE filter (0.45 microns) and a syringe. The filtered solution was added back to a clean 20 mL scintillation vial and re-heated to 40°C. 142.5 mg of succinic acid (1 mole equivalent) was added to the vial. A clear solution was initially observed. After stirring at 40°C for 30 minutes, crystallization of material was observed. The experiment was temperature cycled between 40°C and 5°C in 3-hour cycles for 20 hours. At 5°C, the slurry was filtered using Buchner flask and funnel using grade 1 Whatman filter paper (pore size = 11 µm) under vacuum. The wet cake was washed with 2.5 mL of chilled (5°C) MEK and dried under reduced pressure at 40°C for 2 hours. The dried isolated solid was analysed by XRPD and HPLC. The filtered mother liquor was analysed for concentration by HPLC.

An isolated yield of 78.5 % was achieved, with an HPLC yield of 93.5 % determined based on the mother liquor concentration (see Table 1).

**Table 1: Results from Form 1 succinate salt preparation**

| **Solvent system** | **Input Free Base (mg)** | **Yields (%)** | | **Solid purity % Area** | **Losses to mL's (mg/mL)** | **Losses to wash (mg/mL)** |
|---|---|---|---|---|---|---|
| | | **Theoretical** | **Isolated** | | | |
| Methyl ethyl ketone | 514 | 93.5 | 78.5 | 99.2 | 11.1 | 5.5 |

HPLC indicated a purity of 99.2%; rt=10.65 min.

The XRPD 2Θ diffractogram showed the material to be crystalline (Figure 1) and comprised peaks at diffraction angles of 2θ=3.8° ±0.2° (100%), 10.3° ±0.2° (11.3%), 12.4° ±0.2° (6.8%), 16.2° ±0.2° (23.5%), 17.9° ±0.2° (11.9%), 19.8° ±0.2° (10.2%), 20.4° ±0.2° (13.0%), 23.8° ±0.2° (23.3%) and 26.7° ±0.2° (9.8%) when irradiated with a CuKα light source.

Another batch of *N*-(3-(4-(3-(diisobutylamino)propyl)piperazin-1-yl)propyl)-1H-benzo[d]imidazol-2-amine, sesqui-succinate salt was made and further characterized by HPLC, XRPD and NMR.

HPLC indicated a purity of 98.4%.

XRPD 2Θ diffractogram was identical to that of previously described batch.

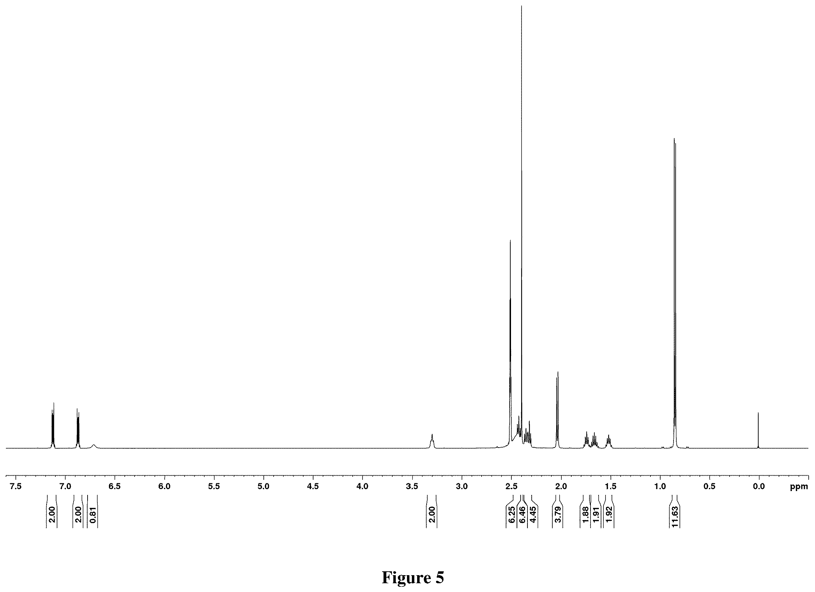

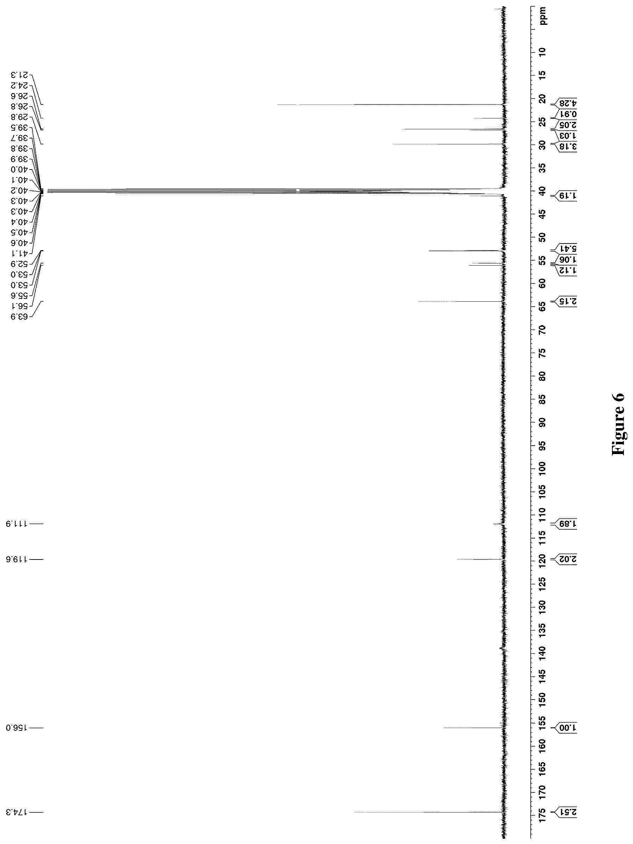

¹H and quantitative ¹³C NMR spectra in DMSO-d₆ are presented in Figure 5 and Figure 6 respectively.

¹H NMR spectrum (500.12 MHz - DMSO-d₆): δ (ppm): 7.13 (2H dd J1 = 6 Hz, J2 = 3 Hz), 6.87 (2H dd J1 = 6 Hz, J2 = 3 Hz), 6.71 (1H br s), 3.30 (2H t J = 7 Hz), 2.42 (6H m, 2.33 4H dt J1 = 16 Hz, J2=7 Hz), 2.04 (4H d J = 7 Hz), 1.74 (2H quintet J = 7 Hz), 1.67 (2H septet J = 7 Hz), 1.52 (2H quintet J = 7Hz), 0.85 (12H d J = 7 Hz) ppm.

Quantitative ¹³C NMR spectrum (121.16 MHz - DMSO-d₆): δ (ppm): 174.3, 156.0, 119.5, 111.9, 63.9 56.1, 55.6, 53.0 (d), 29.8, 26.8, 26.6, 24.2, 21.3. Quantitative ¹³C NMR confirmed the stoichiometry and successful formation of a sesqui-succinate salt.

### BIOLOGY EXAMPLES

The aim of the following study was to assess the neuroprotective effect of *N-*(3-(4-(3-(diisobutylamino)propyl)piperazin-1-yl)propyl)-1*H*-benzo[*d*]imidazol-2-amine) di-sulphate and *N*-(3-(4-(3-(diisobutylamino)propyl)piperazin-1-yl)propyl)-1*H*-benzo[*d*]imidazol-2-amine) sesqui-succinate Form 1 at different concentrations on primary culture of sensory neurons from dorsal root ganglia (DRG) injured by a chemotherapeutic agent: Cisplatin.

### Primary culture of sensory neurons and Schwann cells

Primary sensory neurons (SN) and Schwann cells (SC)were cultured as previously described in Cancer Chem. Pharmacol. 2008 Nov;62(6):995-1007. Briefly, pregnant female rats of 15 days gestation (Rats Wistar; Janvier Labs France) were killed using a deep anesthesia with CO₂ chamber and a cervical dislocation. Then, fetuses were collected and immediately placed in ice-cold L15 Leibovitz medium with a 2% penicillin (10,000 U/mL) and streptomycin (10 mg/mL) solution (PS) and 1% bovine serum albumin (BSA). DRG from spinal cord were treated for 20 min at 37°C with a trypsin-EDTA solution at a final concentration of 0.05% trypsin and 0.02% EDTA. The dissociation was stopped by addition of Dulbecco's modified Eagle's medium (DMEM) with 4.5 g/L of glucose, containing DNAse I grade II (final concentration 0.5 mg/mL) and 10% fetal calf serum (FCS). Cells were mechanically dissociated through the tip of a 10 mL pipette. Cells were then centrifuged at 515 x g for 10 min at 4°C. DMEM F12 with a 1% solution of N2 supplement, 2 mmol/L of L-glutamine, 2% of PS solution, NGF (5 ng/mL) and NT3 (5 ng/mL). Cells were seeded at a density of 15,000 per well in 96-well plates pre-coated with poly-L-lysine and cultured at 37°C in an air (95%)-CO₂ (5%) incubator.

### Compound treatment

On day 14 of culture, primary SN and SC were exposed to the chemotherapeutic agent cisplatin (10 µg/mL, diluted in culture medium) in presence of *N*-(3-(4-(3-(diisobutylamino)propyl)piperazin-1-yl)propyl)-1*H*-benzo[*d*]imidazol-2-amine) di-sulphate or *N*-(3-(4-(3-(diisobutylamino)propyl)piperazin-1-yl)propyl)-1*H*-benzo[*d*]imidazol-2-amine) sesqui-succinate Form for 24h. After 24h, the culture medium was removed (wash-out of Cisplatin) and fresh culture medium with the sulphate salt or the sesqui-succinate salt Form 1 of *N*-(3-(4-(3-(diisobutylamino)propyl)piperazin-1-yl)propyl)-1*H*-benzo[*d*]imidazol-2-amine*)* was added for an additional 24 hours.

### End point evaluation

After 15 days of culture (24h after the cisplatin injury), the cell culture supernatant was collected and the SNs were fixed by a cold solution of ethanol (95%) and acetic acid (5%) for 5 min at -20°C. Cells were washed twice in PBS. Cell membranes were permeabilized and nonspecific sites were blocked with a solution of PBS containing 0.1 % of saponin and 1 % FCS for 15 min at room temperature. Then, cells were incubated for 2h with a mouse monoclonal antibody anti-microtubule associated protein 2 (MAP-2) at dilution of 1/400 in PBS containing 1% fetal calf serum and 0.1% of saponin. This antibody binds specifically MAP-2 present in cell bodies and neurites of all SNs. This antibody was revealed with Alexa Fluor 488 goat antimouse IgG. Nuclei of neurons were labeled by a fluorescent marker (Hoechst solution).

For each condition, 30 pictures (representative of 90% of the well area) per well were automatically taken using ImageXpress (Molecular Devices) with 20x magnification. All images were taken under the same conditions (exposition time, gain and laser intensity). Analysis of different endpoints was automatically performed using Custom Module Editor (Molecular Devices).

The endpoints were:
- Survival of SNs (number of MAP-2 positive SNs)
- Total neurite network (MAP-2): total length of neurites of SNs (expressed in µm)

### Statistical analysis

All values are expressed as mean +/- SEM. Statistical analysis was performed by one-way ANOVA, followed by PLSD Fisher test. Graphs and statistical analyses on the different conditions were performed with GraphPad Prism software version 9.3.1, ^{####}p<0.0001 from control (CTR); *p<0.05; **p<0.01 and ***p<0.001 from cisplatin (10µg/mL) were considered significant.

### Results

### 1- Effect of N-(3-(4-(3-(diisobutylamino)propyl)piperazin-1-yl)propyl)-1H-benzo[d]imidazol-2-amine) di-sulphate

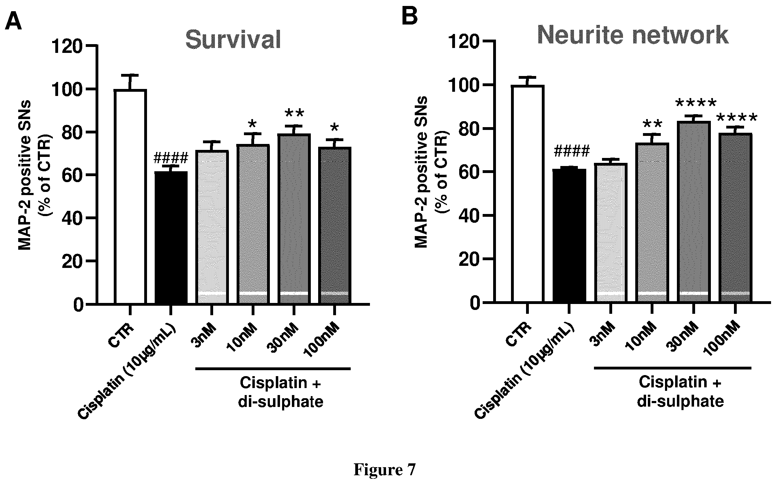

- Sensory neurons survival: as expected, the cisplatin intoxication significantly decreased the SNs survival, as compared to the control group (mean survival: 61%; Figure 7A). Several doses of N-(3-(4-(3-(diisobutylamino)propyl)piperazin-1-yl)propyl)-1H-benzo[*d*]imidazol-2-amine) di-sulphate (3 nM to 100 nM) showed a positive and significant effect on survival, as compared to the cisplatin condition. The maximal effect was obtained with the dose at 30 nM (mean survival: 80%).
- SNs neurite network integrity: cisplatin significantly reduced the SNs neurite network (~61%, Figure 7B). Several doses of N-(3-(4-(3-(diisobutylamino)propyl)piperazin-1-yl)propyl)-1*H*-benzo[*d*]imidazol-2-amine) di-sulphate (3 nM to 100 nM) application were able to improve the neurite network after cisplatin injury, with a maximal effect at 30 nM (mean length: 83%).

### 2- Effect of N-(3-(4-(3-(diisobutylamino)propyl)piperazin-1-yl)propyl)-1H-benzo[d]imidazol-2-amine) sesqui-succinate Form 1

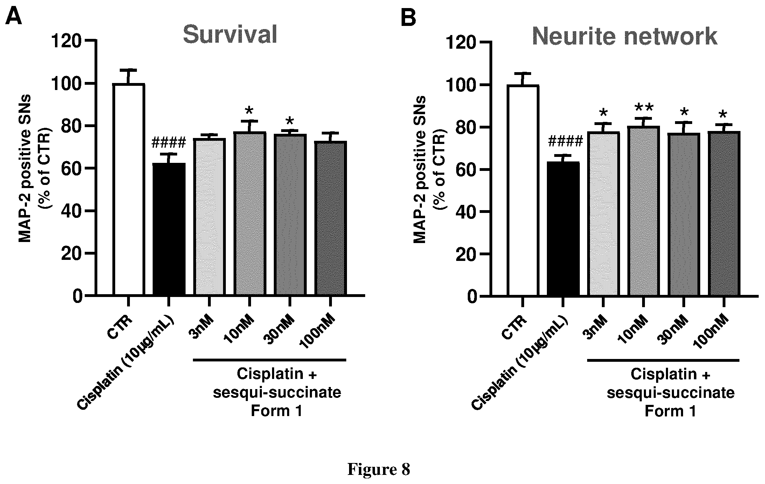

- Sensory neurons survival: as expected, cisplatin significantly decreased SNs survival, as compared to the control group (mean survival: 62%; Figure 8A). Several doses of the sesqui-succinate salt Form 1 (3 nM to 100 nM) displayed positive and significant effect on survival, as compared to the cisplatin condition. The maximal effect was obtained with the dose of sesqui-succinate salt Form 1 at 10 nM (mean survival: ~77%).
- Neurite network integrity: cisplatin strongly reduced the neurite network of SNs (~63%, Figure 8B). All doses from 3 nM to 100 nM of the sesqui-succinate salt Form 1 were able to protect the neurite network from cisplatin damage, with a maximal effect at 10 nM (mean length: ~80%).

## Claims

1. A salt of *N*-(3-(4-(3-(diisobutylamino)propyl)piperazin-1-yl)propyl)-1*H-*benzo[*d*]imidazol-2-amine and pharmaceutically acceptable solvates thereof for use in treating and/or preventing sensory neuron diseases selected from toxic peripheral neuropathies and chemotherapy-induced neuropathies.

2. A salt of *N*-(3-(4-(3-(diisobutylamino)propyl)piperazin-1-yl)propyl)-1*H-*benzo[*d*]imidazol-2-amine and pharmaceutically acceptable solvates thereof for use in delaying in a patient the onset of sensory neuron diseases selected from toxic peripheral neuropathies and chermothe-induced neuropathies.

3. The salt for use according to claim 1 or 2 wherein the salt is selected from the group consisting of *N*-(3-(4-(3-(diisobutylamino)propyl)piperazin-1-yl)propyl)-1*H*-benzo[*d*]imidazol-2-amine sulphate salts and *N*-(3-(4-(3-(diisobutylamino)propyl)piperazin-1-yl)propyl)-1*H*-benzo[*d*]imidazol-2-amine succinate salts, and pharmaceutically acceptable solvates thereof.

4. The salt for use according to claim 3, having Formula I 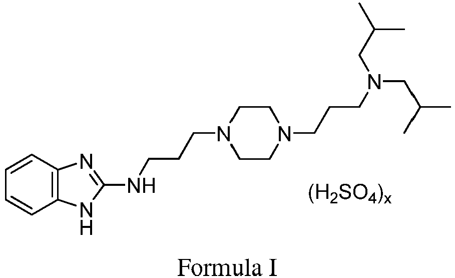 wherein x is 0.5 to 4, and pharmaceutically acceptable solvates thereof.

5. The salt for use according to claim 4 and pharmaceutically acceptable solvates thereof, **characterized in that** x is about 2.

6. The salt for use according to claim 5, wherein the sulphate salt is *N*-(3-(4-(3-(diisobutylamino)propyl)piperazin-1-yl)propyl)-1*H*-benzo[*d*]imidazol-2-amine di-sulphate.

7. The salt for use according to claim 3, having Formula II 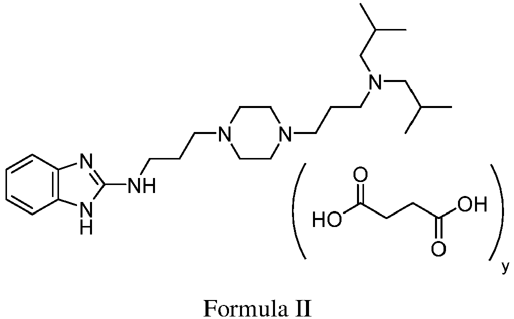 wherein y is 1 to 4, and pharmaceutically acceptable solvates thereof.

8. The salt for use according to claim 7 and pharmaceutically acceptable solvates thereof, **characterized in that** y is about 1.5.

9. The salt for use according to claim 8, wherein the succinate salt is *N*-(3-(4-(3-(diisobutylamino)propyl)piperazin-1-yl)propyl)-1*H*-benzo[*d*]imidazol-2-amine sesqui- succinate.

10. The salt for use according to claim 8, wherein the succinate salt is *N*-(3-(4-(3-(diisobutylamino)propyl)piperazin-1-yl)propyl)-1*H*-benzo[*d*]imidazol-2-amine sesqui- succinate under the crystalline form having an X-ray powder diffraction (XRPD) pattern comprising peaks at diffraction angles of 2θ=3.8° ±0.2°, 10.3° ±0.2°, 12.4° ±0.2°, 16.2° ±0.2°, 17.9° ±0.2°, 19.8° ±0.2°, 20.4° ±0.2°, 23.8° ±0.2° and 26.7° ±0.2° when irradiated with a CuKα light source.

11. The salt for use according to claim 1 or 2, wherein the sensory neuron diseases are selected from chemotherapy-induced neuropathies.

12. The salt for use according to claim 11, wherein the sensory neuron diseases are chemotherapy-induced neuronopathies caused by platinum-based antineoplastic agents.

## Patentansprüche

1. Salz von *N*-(3-(4-(3-(Diisobutylamino)propyl)piperazin-1-yl)propyl)-1*H-*benzo[*d*]imidazol-2-amin und pharmazeutisch annehmbare Solvate davon zur Verwendung bei der Behandlung und/oder Vorbeugung von sensorischen Neuronenerkrankungen, ausgewählt aus toxischen peripheren Neuropathien und Chemotherapie-induzierten Neuropathien.

2. Salz von *N*-(3-(4-(3-(Diisobutylamino)propyl)piperazin-1-yl)propyl)-1*H-*benzo[*d*]imidazol-2-amin und pharmazeutisch annehmbare Solvate davon zur Verwendung bei der Verzögerung des Ausbruchs von sensorischen Neuronenerkrankungen bei einem Patienten, ausgewählt aus toxischen peripheren Neuropathien und Chemotherapie-induzierten Neuropathien.

3. Salz zur Verwendung nach Anspruch 1 oder 2, wobei das Salz ausgewählt ist aus der Gruppe bestehend aus *N*-(3-(4-(3-(Diisobutylamino)propyl)piperazin-1-yl)propyl)-1*H-*benzo[*d*]imidazol-2-amin-sulfatsalzen und *N*-(3-(4-(3-(Diisobutylamino)propyl)piperazin-1-yl)propyl)-1*H*-benzo[*d*]imidazol-2-amin-succinatsalzen, und pharmazeutisch annehmbaren Solvaten davon.

4. Salz zur Verwendung nach Anspruch 3, welches die Formel I aufweist 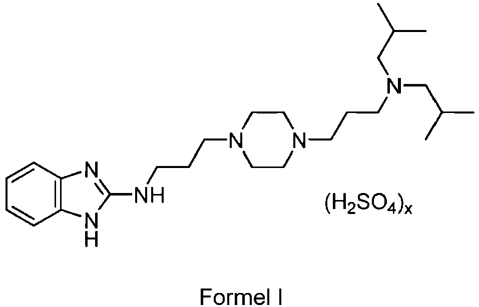 wobei x 0,5 bis 4 beträgt, und pharmazeutisch annehmbare Solvate davon.

5. Salz zur Verwendung nach Anspruch 4 und pharmazeutisch annehmbare Solvate davon, **dadurch gekennzeichnet, dass** x etwa 2 beträgt.

6. Salz zur Verwendung nach Anspruch 5, wobei das Sulfatsalz *N*-(3-(4-(3-(Diisobutylamino)propyl)piperazin-1-yl)propyl)-1*H*-benzo[*d*]imidazol-2-amin-disulfat ist.

7. Salz zur Verwendung nach Anspruch 3, welches die Formel II aufweist, 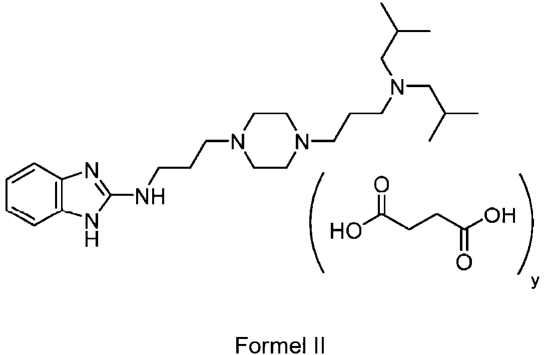 wobei y 1 bis 4 beträgt, und pharmazeutisch annehmbare Solvate davon.

8. Salz zur Verwendung nach Anspruch 7 und pharmazeutisch annehmbare Solvate davon, **dadurch gekennzeichnet, dass** y etwa 1,5 beträgt.

9. Salz zur Verwendung nach Anspruch 8, wobei das Succinatsalz *N*-(3-(4-(3-(Diisobutylamino)propyl)piperazin-1-yl)propyl)-1*H*-benzo[*d*]imidazol-2-amin-sesquisuccinat ist.

10. Salz zur Verwendung nach Anspruch 8, wobei das Succinatsalz *N*-(3-(4-(3-(Diisobutylamino)propyl)piperazin-1-yl)propyl)-1*H*-benzo[*d*]imidazol-2-amin-sesquisuccinat in der kristallinen Form ist, welche ein Röntgenpulverdiffraktionsmuster (XRPD) aufweist, welches Peaks bei Diffraktionswinkeln von 2θ=3,8° ±0,2°, 10,3° ±0,2°, 12,4 ° ±0,2°, 16,2° ±0,2°, 17,9° ±0,2°, 19,8° ±0,2°, 20,4° ±0,2°, 23,8° ±0,2° und 26,7° ±0,2° umfasst, wenn mit einer CuKα-Lichtquelle bestrahlt.

11. Salz zur Verwendung nach Anspruch 1 oder 2, wobei die sensorischen Neuronenerkrankungen ausgewählt sind aus Chemotherapie-induzierten Neuropathien.

12. Salz zur Verwendung nach Anspruch 11, wobei die sensorischen Neuronenerkrankungen Chemotherapie-induzierte Neuronopathien sind, welche durch platinbasierte Antineoplastika verursacht sind.

## Revendications

1. Sel de *N*-(3-(4-(3-(diisobutylamino)propyl)pipérazin-1-yl)propyl)-1*H-*benzo[*d*]imidazol-2-amine et solvates pharmaceutiquement acceptables de celui-ci pour une utilisation dans le traitement et/ou la prévention de maladies des neurones sensoriels sélectionnées parmi des neuropathies périphériques toxiques et des neuropathies induites par chimiothérapie.

2. Sel de *N*-(3-(4-(3-(diisobutylamino)propyl)pipérazin-1-yl)propyl)-1*H-*benzo[*d*]imidazol-2-amine et solvates pharmaceutiquement acceptables de celui-ci pour une utilisation afin de retarder chez un patient le déclenchement de maladies des neurones sensoriels sélectionnées parmi des neuropathies périphériques toxiques et des neuropathies induites par chimiothérapie.

3. Sel pour une utilisation selon la revendication 1 ou 2, dans lequel le sel est sélectionné dans le groupe consistant en des sels de sulfate de *N*-(3-(4-(3-(diisobutylamino)propyl)pipérazin-1-yl)propyl)-1*H*-benzo[*d*]imidazol-2-amine et des sels de succinate de *N*-(3-(4-(3-(diisobutylamino)propyl)pipérazin-1-yl)propyl)-1*H-*benzo[d]imidazol-2-amine, et solvates pharmaceutiquement acceptables de celui-ci.

4. Sel pour une utilisation selon la revendication 3, de Formule I 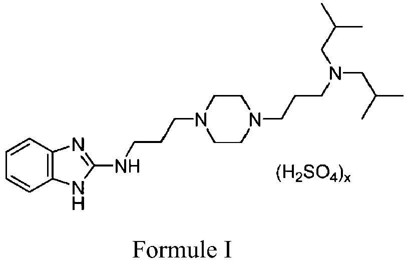 dans laquelle **x** est de 0,5 à 4, et solvates pharmaceutiquement acceptables de celui-ci.

5. Sel pour une utilisation selon la revendication 4 et solvates pharmaceutiquement acceptables de celui-ci, **caractérisés en ce que x** est d'environ 2.

6. Sel pour une utilisation selon la revendication 5, dans lequel le sel de sulfate est le disulfate de *N*-(3-(4-(3-(diisobutylamino)propyl)pipérazin-1-yl)propyl)-1*H-*benzo[*d*]imidazol-2-amine.

7. Sel pour une utilisation selon la revendication 3, de Formule II 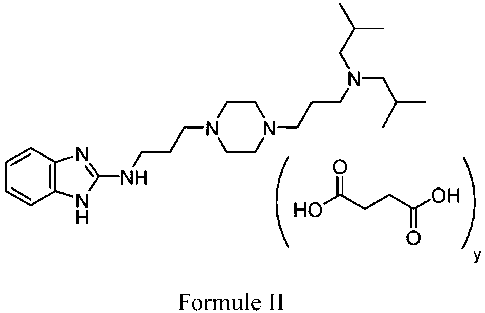 dans laquelle y est de 1 à 4, et solvates pharmaceutiquement acceptables de celui-ci.

8. Sel pour une utilisation selon la revendication 7 et solvates pharmaceutiquement acceptables de celui-ci, **caractérisés en ce que** y est d'environ 1,5.

9. Sel pour une utilisation selon la revendication 8, dans lequel le sel de succinate est le sesqui-succinate de *N*-(3-(4-(3-(diisobutylamino)propyl)pipérazin-1-yl)propyl)-1*H-*benzo[*d*]imidazol-2-amine.

10. Sel pour une utilisation selon la revendication 8, dans lequel le sel de succinate est le sesqui-succinate de *N*-(3-(4-(3-(diisobutylamino)propyl)pipérazin-1-yl)propyl)-1*H-*benzo[*d*]imidazol-2-amine sous la forme cristalline ayant un motif de diffraction sur poudre de rayons X (XRPD) comprenant des pics à des angles de diffraction de 2θ = 3,8° ± 0,2°, 10,3° ± 0,2°, 12,4° ± 0,2°, 16,2° ± 0,2°, 17,9° ± 0,2°, 19,8° ± 0,2°, 20,4° ± 0,2°, 23,8° ± 0,2° et 26,7° ± 0,2° lorsqu'il est irradié avec une source de lumière CuKα.

11. Sel pour une utilisation selon la revendication 1 ou 2, dans lequel les maladies des neurones sensoriels sont sélectionnées parmi des neuropathies induites par chimiothérapie.

12. Sel pour une utilisation selon la revendication 11, dans lequel les maladies des neurones sensoriels sont des neuronopathies induites par chimiothérapie causées par des agents antinéoplasiques à base de platine.
